# EUROPEAN PATENT APPLICATION

(11) **EP 3 015 069 A1**
(43) Date of publication of application: **04.05.2016**
(21) Application number: 14191088.5
(22) Date of filing: 30.10.2014
(51) Int. Cl.: A61B 8/00

(54) **Apparatus for suspended ultrasonography and corresponding method**

(71) Applicant: Universitätsklinikum Jena, 07743 Jena (DE); eZono AG, 07743 Jena (DE)
(72) Inventor: Norgauer, Johannes, 07743 Jena (DE); Sobrino, Eliseo, 07749 Jena (DE)
(74) Representative: Schulz Junghans Patentanwälte PartGmbB

(57) **Abstract**

The invention deals with an apparatus (1) for suspended ultrasonography and corresponding method, wherein the apparatus comprises at least:
- a tank (2) for retaining an impedance-matching liquid (3), into which a body part (4) is insertable to be at least partially covered by the liquid (3);
- at least one ultrasonic transducer (5) to emit an ultrasound wave (6) and to receive a reflected ultrasound wave (7); and
- at least one moveable manipulator (8) for holding and guiding the at least one ultrasonic transducer (5),

wherein the at least one ultrasonic transducer (5) is arranged to produce images of a covered portion (9) of the respective body part (4) from at least a predetermined distance (10) without inducing pressure on the respective body part (4).

The proposed invention makes it very much easier to carry out suspension sonography and, moreover, a remarkable abbreviation of the required examination time is achieved.

## Description

This invention deals with an apparatus for suspended ultrasonography and a corresponding method of guiding a manipulator of an apparatus for suspended ultrasonography, in particular for diagnosis and monitoring altered microcirculation, integrity of different structures like bones or tendons and/or oedema formation in tissues. These pathophysiologicals signs are common effects in inflammatory conditions and cancer; e.g. various autoimmunological or autoinflammatory diseases such as lupus eryrthematodes, mixed connective tissue disease, systemic sclerosis, dermatomyositis, primary Raynaud's syndrome, and psoriasis-arthritis. Ankylosing spondylitis, enteropathic arthritis, and reactive spondyloarthritis (infection with Chlamydia trachomatis, Shigella flexneri, Salmonella spp, Yersinia enterolytica, Campylobacter fetus jejuni, Clostridium difficile) show pronounced vascular, structural-dependent and neural-dependent regulative abnormalities in the skin, tendons or entheseal structures. The features of these diseases are often overlapping and require multiple diagnostic methods.

Vascular abnormalities with morphological and functional alterations in the nail fold of the hand or in the extremities by patients with lupus eryrthematodes, mixed connective tissue disease, systemic sclerosis, dermatomyositis, primary Raynaud's syndrome can he monitored semi-quantitatively by capillary-microscopy or thermography, respectively, as for example in the US 6,555,320. Both methods are poorly standardized and require highly skilled and trained specialists. Moreover, conventional thermography can only be monitored under standardized special conditions requiring a temperature-controlled and humidity-controlled environment. Further, indirect biochemical methods are known based on the examination of blood, such as described in the US 7,807,389, the US 7,605,166 and the US 6,589,755.

Moreover, tumours require a blood supply to grow and the blood and lymph vessels provide a route for the spread of tumour cells to other sites in the body. In order to facilitate oxygen supply of growing tumours, tumour cells or cells in the surrounding extracellular matrix alter the permeability of vessels allowing extravasation of cells and fluid from the vessels forming local oedema formation. Therefore, detecting altered microcirculation or impaired blood flow in vessels as well as oedema formation might help to detect micrometastasis or primary tumours at very early stage. In addition, detecting central blood vessels in the context of tumours or metastasis might be the initial step of treatment. These strategies have the added advantages that preventing blood flow through a single vessel can kill a large number of tumour cells and that delivering vascular-damaging drugs to their site of action on the blood vessels is much easier than delivery directly to the tumour cells. Since such diagnostic and therapeutic procedures for cancer have to provide stable, controllable and reproducible monitoring conditions without the application of pressure.

For diagnosis and monitoring of inflammation in tissue or cancer, e.g. entheseal inflammation of spondyloarthropathies such psoriasis-arthritis, ankylosing spondylitis, enteropathic arthritis, reactive spondyloarthritis, as well as cancer, such as breast cancer, currently the gold standard is to use magnetic resonance imaging (MRI), as for example described in the US 7,660,619. The problem with this method is that it can only monitor morphological alterations and oedema as secondary sign. Second, MRI cannot image blood flow in a small vessel formation as inflammatory sign or tumour nutrient pathway. Third, MRI is very expensive. In the emerging technology using fluoroscopy for monitoring vessel formation, for example as for example described in the US 7,879,314, is also expensive and is unable to resolve morphological structures. These techniques involve injecting chemicals into the body with side effects. Since these techniques require local and transient accumulation of dye, multiple injections are necessary for analysis of different extremities and injections in the lower extremities burden a high risk.

Conventional ultrasound has also been used for imaging of inflammation and tumour cells, e.g. in spondyloarthropathies or breast cancer. However, the B-mode (brightness modulation) can only, similar to MRI, detect tissue structure, bone integrity and oedema formation. Moreover, hypodense structures cannot only be caused by oedema but also by an artificial effect called anisotropy. Discrimination between these two effects is often difficult and time consuming. Therefore, suspended ultrasound (no direct probe contact to skin, but contact through a thick gel layer over the skin without generating mechanical pressure to the analysed tissue) has been studied at two locations: Ancona (Prof. Grassi) and Jena (Prof. Norgauer). This technique shows promising results with respect to localization and analysis of vascular structures and dynamics. However, the scanning process is extremely time consuming and requires about three hours in order to analyse hands, Achilles tendon and the region around the patella. These regions have to be scanned to achieve reliable data. It requires highly skilled staff and it is ergonomically incorrect for the operator. Furthermore, repeatability and reproducibility of the procedure is open to human error. The known methods have the disadvantage that they are time-consuming, expensive and/or have an unsatisfactory resolution for display of the inflamed vascular structures.

The problems raised in the known prior art will be at least partially solved in the invention as described below. The features according to the invention are specified within the independent claims, advantageous alternative embodiments of which will be shown in the dependent claims. The features of the claims can be combined in any technically meaningful way, and the explanations from the following specification as well as features from the figures which show additional embodiments of the invention can be considered.

The invention deals with an apparatus for suspended ultrasonography, which comprises at least:
- a tank for retaining an impedance-matching liquid, into which a body part is insertable to be at least partially covered by the liquid;
- at least one ultrasonic transducer to emit an ultrasound wave and to receive a reflected ultrasound wave; and
- at least one moveable manipulator for holding and guiding the at least one ultrasonic transducer,
wherein the at least one ultrasonic transducer is arranged to produce images of a covered portion of the respective body part from at least a predetermined distance without inducing pressure on the respective body part.

The apparatus described in here is arranged to support the diagnosis and monitoring of above described diseases by applying the method of suspended ultrasonography, also known as suspension sonography. The suspended ultrasonography carried out by hand is, as mentioned above, exhausting and cumbersome. The ultrasonic transducer has to be held in a distant position and cannot rest on the human body, as this is the case when using conventional ultrasonography. Therefore, carrying out this method by hand is quite tiring. In addition, it requires high experience and takes at least three hours. To the contrary, this proposed apparatus allows a more ergonomic conduction of this superior diagnosis and monitoring method.

At first, a tank for retaining an impedance-matching liquid is provided into which a body part, for example a hand (in particular the nail folds), a foot (in particular the Achilles tendon), a knee (in particular the patella), a breast or other body part, which is to be screened, can be inserted. This body part is at least inserted in such a way, that at least that portion of the body part which is to be screened is covered by the impedance-matching liquid in the tank. Impedance-matching is a liquid which matches the transducer-impedance of the body (part) to be screened. For a convenient and ergonomic examination procedure for the patient a support, fixture or gripping device may be placed in and/or at the tank so that the patient can insert his respective body part in such a way that the body part maintains a relaxed state whilst still in the tank. However, this might not be required in any case, because the imaging by the transducer can be carried out quite fast. Further, at least one ultrasonic transducer is provided to emit the required ultrasound wave and to receive the reflected ultrasound wave for generating the signals to be prepared as the user is used to, for example a physician. Therefore, the ultrasonic transducer may be a prior art transducer as well as the apparatus and computation for preparation of the signals.

In addition, the ultrasonic transducer is held and guided by a movable manipulator, which is arranged to bring the ultrasonic transducer into any position required for carrying out the suspended ultrasonography. Of course, the emitting and receiving portion of the ultrasonic transducer during the imaging is placed into the impedance-matching liquid to produce good signals. The manipulator in one advantageous embodiment is a robot arm automatically actuated, which is, for example, power assisted and/or may be moved by a joystick by a user, for example a physician or a technician. In a preferred embodiment the robot arm moves automatically in a predefined pattern that covers the area of interest without the need of an operator. In another advantageous embodiment the manipulator is an articulate mechanical arm transforming the ergonomic user's hand movement into the required movement. In the latter case, at least the ergonomic requirement can be adapted to be less exhausting for the user.

During the carrying out of the method, the at least one ultrasonic transducer is kept in a predetermined distance to the portion of the respective body part to be imaged. That means the ultrasonic transducer never rests on the portion of the respective body part to be imaged, but a thick layer of impedance-matching liquid is placed between the transducer and the portion of the respective body part, so that neither weight nor movement of the at least one ultrasonic transducer is transformed into pressure on the portion of the respective body part. Therefore, the vascular structures in the portion of the respective body part are not exposed to external pressure and, thus, do not collapse caused by the examination, as this is the case in conventional ultrasonography.

The predetermined distance is dependent on the mechanical properties of the impedance-matching liquid, which preferably is a conventional ultrasonographic water-gel. For such a conventional ultrasonographic liquid, the distance may be 2 mm to 50 mm [millimetres]. The predetermined distance is to be arranged between a minimum distance for avoiding induction of pressure and a maximum distance for achieving good quality images. Therefore, the predetermined distance should be chosen spaced from the required minimum distance, so that pressure influence is securely avoided. However, the choice of predetermined distance should also be made under consideration of the accuracy of the movable manipulator, of the control and of the power assistance of the movable manipulator and/or of the resolution of the ultrasonic transducer.

According to a preferred embodiment of the apparatus, the temperature of the liquid in the tank is controlled, wherein preferably the liquid is continuously exchanged.

By controlling the temperature, an undercooling, or at least a undesirable cooling down, and/or heating-up of the respective body part can be prevented so that the body part is monitored under satisfactorily repeatable conditions without the collapse of vascular structures caused by temperature drop and, to the opposite, expansion of the vascular structures due to rising temperature, respectively. In a preferred embodiment, the liquid is controlled at a temperature which simulates a normal environment, as for example the good thermal insulator air at 20° C. This mode can be called temperature-neutral as the body part does not need to heat or cool the body part more or less than, eventually clothed, in normal air environment.

This method also shows the advantage that for the liquid environment of the body part the temperature of the body part is fast and easily brought into a desired condition as the heat exchange between the body part and a liquid is better than exposed to air. Very preferably, the liquid is continuously exchanged to avoid heating up of the liquid in the near environment of the body part. In particular, the liquid can be chosen much more viscous as the support properties for conventional ultrasonography is not required for the guiding of the ultrasonic transducer by the movable manipulator. Therefore, the liquid can be easily exchanged, for example by a pump.

In another preferable embodiment of the apparatus, the temperature of the liquid is altered, preferably by exchanging the liquid.

In this embodiment, the behaviour of the vascular structures in the portion of the body part to be imaged can be observed and, therefore, the degree of inflammation and impairment to the tissue can be monitored more easily and under controlled repeatable conditions. As explained according to above embodiment with the temperature control, the easiest and fastest way to alter the temperature is to exchange the liquid. However, in addition or alternatively the liquid may be heated within the tank and cooled down, respectively, by an integral heater and cooler, respectively.

According to another preferred embodiment of the apparatus, the apparatus further comprises at least one temperature probe and the temperature of at least the currently examined portion of the respective body part is determinable by the at least one temperature probe.

In this preferred embodiment of the apparatus, a temperature probe is provided to measure and observe the temperature of that currently examined portion of the body part, so that this information can be combined with the observation by the ultrasonic transducer. Additionally, the temperature of the entire body part may be observed. Therefore, this method broadens the possibility of diagnosis of the vascular structure, in particular in combination with a temperature guiding of the liquid. New dynamic aspects as the behaviour, in particular the speed of the adaption of the vascular structure, to the change of the liquid temperature can be observed. The probe may be of any kind. Preferably the temperature probe is an infrared camera making images of the heat radiation of the portion to be imaged.

According to another advantageous embodiment of the apparatus the manipulator further comprises a distance sensor and the ultrasonic transducer is kept at a predetermined distance based on distance data from the distance sensor.

In this preferred embodiment of the apparatus further a distance sensor is provided, which measures the distance between at least one ultrasonic transducer and the portion of the body part. Preferably, the entire body part inserted into the tank is kept in distance to the at least one ultrasonic transducer and the manipulator, so that influences by contact between the body part and the apparatus is avoided. The distance sensor may be an additional sensor, wherein the distance is measured without contact. The sensing may be carried out by optical devices, for example a laser measurement, but preferably the distance sensor is provided integrally into at least one of the ultrasonic transducers, so that the same data which is used for imaging can be used for providing distance data and keeping at least one ultrasonic transducer in the predetermined distance from the portion of the respective body part. In a preferred embodiment, the sensing is automatically carried out by a feedback calculation from the ultrasound image, in which the first reflection received is that of the skin and so distance between transducer and patient can be measured in real time in the image itself.

According to another advantageous embodiment of the apparatus the apparatus further comprises at least one display, wherein on the display prepared image data generated by the ultrasonic transducer is displayed in 2.5D, preferably in 3D, wherein preferably the image data is annotatable by a user, preferably via at least one touch-screen display.

For a good preparation of the generated image data, the image data is displayed in 2.5D or 3D on a display for a user. Therefore, the user can fly through images and observe the condition of the vascular structure. Very preferably, the user can make annotations at the structures observed, so that at a later point of time the user or another user can easily find back into the reasons for her or his diagnosis. In particular, the user can compare image data from an earlier point of time with younger image data, which was made to monitor the condition of the patient. Preferably, the annotations can be made via a touch-screen display, so that the structures observed by the user can be easily marked-up. Very preferably, the user is assisted by a structure-recognition tool automatically recognizing boundary surfaces. According to another aspect of the invention, a method of guiding a manipulator of an apparatus according to the above specification is proposed. That manipulator holds and guides an ultrasonic transducer to carry out suspended ultrasonography, wherein the ultrasonic transducer is guided by the manipulator to generate images of at least one portion of a body part, which body part is inserted into the tank and covered by the impedance-matching liquid, wherein the distance of the ultrasonic transducer to at least the covered portion of the respective body part, of which images are being generated, is determined and a signal is emitted at the latest when the predetermined distance is underrun.

In this method, the at least one ultrasonic transducer is guided by the manipulator and the distance to the portion of the respective body part which is to be imaged is observed and a signal is emitted at the latest when the predetermined distance is underrun. For cautious measurements in one embodiment there is a pre-limit which triggers the signal or the signal intensity is raised from that pre-limit until the predetermined distance. Thereby it is easy to avoid that the at least one ultrasonic transducer comes into pressure inducing contact with the portion of the respective body part to be imaged. The signal emitted can be a light signal and/or a sound signal for the user, so that the user can react to it. Very preferably, when the user is moving the manipulator via a joystick, the user gets a signal by a rumble pack and/or by an increased resistance, so that the user naturally knows in an ergonomic way how to move the manipulator by the joystick.

According to another advantageous embodiment of the method described above, the method further comprises at least the following steps: guiding the ultrasonic transducer by the manipulator to generate images of a covered portion of the respective body part without inducing external pressure on the respective body part by keeping the ultrasonic transducer at least at the predetermined distance from at least the covered portion of the respective body part, of which portion images are being generated, based at least on the emitted signal.

In this embodiment the manipulator is moved automatically and the signal is a, preferably electric, signal for a control circuit guiding the manipulator at the predetermined distance. Such a method can be carried out autonomic and very fast and exact.

According to another advantageous embodiment of the method according to above specification the at least one portion of the respective body part imaged by the method is locally and/or chronologically superimposed with other information, such as:
- temperature information by at least one infrared camera;
- image information from a previously conducted computed tomography and/or magnet resonance imaging;
- image information by angiography.

By superimposing the images made by the method described above, the information for the user is remarkably enhanced. In particular, the local and chronological superimposition gives the user, for example a physician, very good data to make a diagnosis (of changes).

According to another advantageous embodiment of the method described above the produced image data is prepared to be displayed as 2.5D-images, preferably 3D-images, on a display for a user, preferably in real-time, wherein preferably the data is stored with the additionally gathered information.

For a good preparation of the generated image data, the image data is displayed in 2.5D or 3D on a display for a user. Therefore, the user can fly through images and observe the condition of the vascular structure. Very preferably, the user is assisted by a structure-recognition tool automatically recognizing boundary surfaces.

According to another advantageous embodiment of the method described above the image data is annotatable by a user, preferably by use of a touch-screen display.

According to this embodiment, the user can make annotations at the structures observed, so that at a later point of time the user or another user can easily find back into the reasons for her or his diagnosis. In particular, the user can compare image data from an earlier point of time with younger image data, which was made to monitor the condition of the patient. Preferably, the annotations can be made via a touch-screen display, so that the structures observed by the user can be easily marked-up.

According to another aspect of the invention, a computer program is proposed, which is implemented to carry out the method according to above specification, wherein the computer program is executable on a computer comprising at least one central processing unit, which computer is comprised in an apparatus according to any embodiment of above specification.

The computer program can be written in any useful software-language and makes use of the required components of a computer, such a central processing unit, graphical processor, working storage and/or solid storage. It may be implementable on a working station providing a user surface or into machine code or by individual circuit board architecture.

According to another aspect of the invention, a computer program product comprising a computer program according to above specification, wherein the computer program is executable when the computer program product is read by and installed on a computer comprising at least one central processing unit, which computer is comprised in an apparatus according to any embodiment of above specification.

The computer program product as solid storage includes the above described computer program. Useful computer program products are USB storages with flash memory, a CD-ROM or DVD, a disk or data tape. It may also be physical storage place in a server accessible by a user to download for installation of the computer program on her or his device.

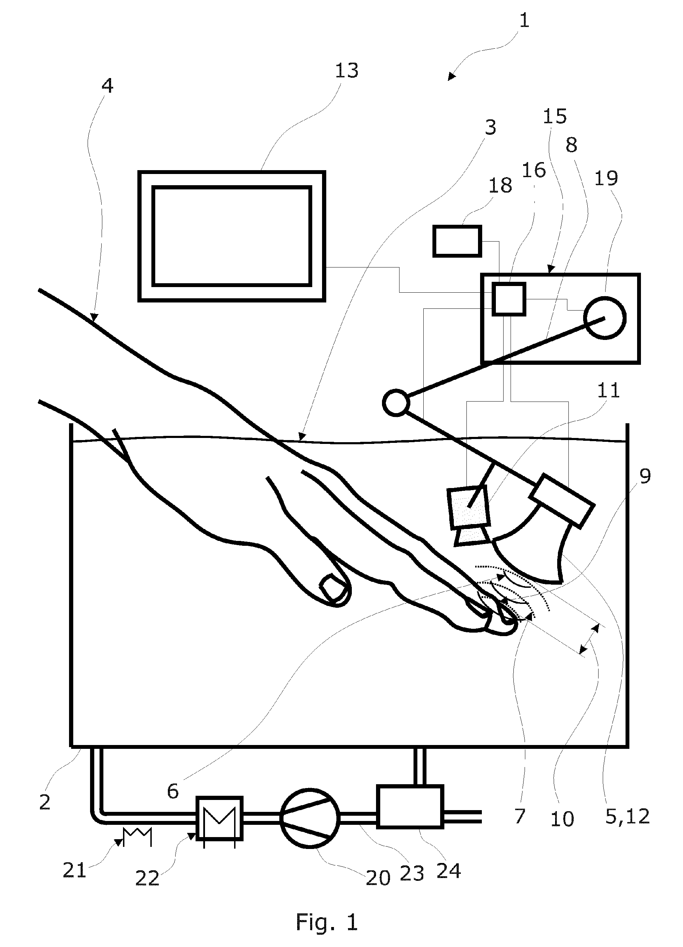

The invention according to above description will be explained in detail based on the technical background, wherein reference is made to the corresponding drawing, which shows a preferred embodiment. The invention is not limited by that solely schematic drawing in any way. It is to be noted that the drawing is not to scale and cannot be considered for concluding proportions. It is shown in
- Fig. 1:: a preferred embodiment of the apparatus for automated suspended ultrasonography.

In Fig. 1 is shown an apparatus 1 for a human body part 4, here a hand, to be imaged by suspended ultrasonography. The body part 4 is partially inserted into a tank 2 filled with an impedance-matching liquid 3. The impedance-matching liquid 3 is exchangeable by the pump 20. So via the pipe 23 and the reservoir 24 the impedance-matching liquid 3 can be renewed, heated up by the heater 21, cooled down by the cooler 22 and/or maintained at a desired temperature. The temperature of the impedance-matching liquid 3 may be measured by the temperature probe 11, which is an infrared camera here, and/or by an additional temperature probe (not shown). The covered portion 9, preferably only at least one of the nail folds, of the body part 4 is exposed to ultrasound waves 6 emitted by the ultrasonic transducer 5 and the elements of that portion 9 reflect the ultrasound waves 6 at least partially, which reflected ultrasound waves 7 are received by the ultrasonic transducer 5. As can be seen in Fig. 1 the ultrasonic transducer 5 is spaced from the portion 9 at a predetermined distance 10. Therefore, no pressure is induced into the portion 9 from the weight and or movement of the ultrasonic transducer 9.

In Fig. 1, a temperature probe 11, here an infrared camera, observes the temperature of the portion 9 giving additional information on the situation of the vascular structure inside the portion 9. The manipulator 8 in this embodiment is actuated autonomic by a central processing unit 16 controlling the at least one actuator 19 of the manipulator 8. Further, in the embodiment the ultrasonic transducer 5 is also used as a distance sensor 12, which sends data to the central processing unit 16. In the end, preferably in real-time, the generated data is prepared for and transmitted to a display for interaction with a user, for example a physician. Preferably, the display 13 is provided with a touch-screen and allows input by the user. Preferably, the images are superimposed with data from another imaging method, such as MRI and angiography. Here, the other image data is provided by an image data source 18.

The proposed invention makes it very much easier to carry out suspension sonography and, moreover, a remarkable abbreviation of the required examination time is achieved.

### list of reference numerals

- 1: apparatus
- 2: tank
- 3: impedance-matching liquid
- 4: body part
- 5: ultrasonic transducer
- 6: ultrasound wave
- 7: reflected ultrasound wave
- 8: manipulator
- 9: covered portion
- 10: predetermined distance
- 11: temperature probe
- 12: distance sensor
- 13: display

- 15: computer
- 16: central processing unit

- 18: image data source
- 19: actuator
- 20: pump
- 21: heater
- 22: cooler
- 23: pipe
- 24: reservoir

## Claims

1. Apparatus (1) for suspended ultrasonography, comprising at least:
- a tank (2) for retaining an impedance-matching liquid (3), into which a body part (4) is insertable to be at least partially covered by the liquid (3);
- at least one ultrasonic transducer (5) to emit an ultrasound wave (6) and to receive a reflected ultrasound wave (7); and
- at least one moveable manipulator (8) for holding and guiding the at least one ultrasonic transducer (5),
wherein the at least one ultrasonic transducer (5) is arranged to produce images of a covered portion (9) of the respective body part (4) from at least a predetermined distance (10) without inducing pressure on the respective body part (4).

2. Apparatus (1) according to claim 1, wherein the temperature of the liquid (3) in the tank (2) is controlled, wherein preferably the liquid (3) is continuously exchanged.

3. Apparatus (1) according to claim 2, wherein the temperature of the liquid (3) is altered, preferably by exchanging the liquid (3).

4. Apparatus (1) according to any of the preceding claims, wherein the apparatus (1) further comprises at least one temperature probe (11) and the temperature of at least the currently examined portion (9) of the respective body part (4) is determinable by the at least one temperature probe (11).

5. Apparatus (1) according to any of the preceding claims, wherein the manipulator (8) further comprises a distance sensor (12) and the ultrasonic transducer (5) is kept at a predetermined distance (10) based on distance data from the distance sensor (12).

6. Apparatus (1) according to any of the preceding claims, wherein the apparatus (1) further comprises at least one display (13), wherein on the display (13) prepared image data generated by the ultrasonic transducer (5) is displayed in 2.5D, preferably in 3D, wherein preferably the image data is annotatable by a user, preferably via at least one touch-screen display (13).

7. Method of guiding a manipulator (8) of an apparatus (1) according to any of claims 1 to 6, which manipulator (8) holds and guides an ultrasonic transducer (5) to carry out suspended ultrasonography, wherein the ultrasonic transducer (5) is guided by the manipulator (8) to generate images of at least one portion (9) of a body part (4), which body part (4) is inserted into the tank (2) and covered by the impedance-matching liquid (3), wherein the distance (10) of the ultrasonic transducer (5) to at least the covered portion (9) of the respective body part (4), of which images are being generated, is determined and a signal is emitted at the latest when the predetermined distance (10) is underrun.

8. Method according to claim 7, further comprising at least the following steps:
guiding the ultrasonic transducer (5) by the manipulator (8) to generate images of a covered portion (9) of the respective body part (4) without inducing external pressure on the respective body part (4) by keeping the ultrasonic transducer (5) at least at the predetermined distance (10) from at least the covered portion (9) of the respective body part (4), of which portion (9) images are being generated, based at least on the emitted signal.

9. Method according to claim 7 or 8, wherein the at least one portion (9) of the respective body part (4) imaged by the method is locally and/or chronologically superimposed with other information, such as:
- temperature information by at least one infrared camera;
- image information from a previously conducted computed tomography and/or magnet resonance imaging;
- image information by angiography.

10. Method according to any of the claims 7 to 9, wherein the produced image data is prepared to be displayed as 2.5D-images, preferably 3D-images, on a display (13) for a user, preferably in real-time, wherein preferably the data is stored with the additionally gathered information.

11. Method according to claim 10, wherein the image data is annotatable by a user, preferably by use of a touch-screen display (13).

12. Computer program implemented to carry out the method according to any of the claims 7 to 11, wherein the computer program is executable on a computer (15) comprising at least one central processing unit (16), which computer (15) is comprised in an apparatus (1) according to any of the claims 1 to 6.

13. Computer program product comprising a computer program according to claim 12, wherein the computer program is executable when the computer program product is read by and installed on a computer (15) comprising at least one central processing unit (16), which computer (15) is comprised in an apparatus (1) according to any of the claims 1 to 6.
